# EUROPEAN PATENT SPECIFICATION

(11) **EP 3 395 398 B1**
(45) Date of publication and mention of the grant of the patent: **22.09.2021**
(21) Application number: 17193053.0
(22) Date of filing: 26.09.2017
(51) Int. Cl.: A61N 1/04, A61N 1/36, A61N 1/32

(54) **MUSCLE ELECTROSTIMULATION DEVICE**
MUSKELELEKTROSTIMULATIONSVORRICHTUNG
DISPOSITIF D'ÉLECTROSTIMULATION MUSCULAIRE

(30) Priority: 27.04.2017 JP 2017088039
(43) Date of publication of application: 31.10.2018
(73) Proprietor: MTG Co., Ltd., Nagoya Aichi 453-0041 (JP)
(72) Inventor: MATSUSHITA, Tsuyoshi, Nagoya, Aichi 453-0041 (JP)
(74) Representative: Noble, Nicholas

(56) References cited:
- EP-A1- 0 603 452
- WO-A1-00/41764
- WO-A2-2006/113802
- GB-A- 2 504 984
- US-B1- 6 341 237

## Description

The present invention relates to muscle electrostimulation devices.

A muscle electrostimulation device is known as one example of devices for training muscle. A muscle electrostimulation device causes an electric current to flow in the muscle fiber to stress or relax the muscle. This is expected to grow the muscle. For example, a muscle electrostimulation device described in patent document 1 is proposed. The muscle electrostimulation device described in patent document 1 is attached to the middle of the abdomen and mainly stimulates the abdominal rectus muscle.

[patent document 1] JP2017-6644

US6341237 is directed to a device for administrating electro-muscle stimulation including a flexible covering having a plurality of spaced apart electrodes. The flexible covering is shaped like a belt and is designed to encircle and be connected around a portion of a patient's body. The belt is fabricated from an elastic material so that the electrodes are pressed against the skin of the patient to promote better electrical conduction. The electrodes are selectively positionable to different locations on the belt, so they may be placed directly over a selected muscle or muscle group. Each electrode has its own individual control for adjusting the level of the electrical stimulation signal, so that different muscles can receive different levels of stimulation, and the level of stimulation may be changed during the course of treatment. A master adjustment control is used to adjust the stimulation signal level applied to all the electrodes. The individual adjustment controls are located adjacent their respective electrodes on the belt.

WO 2006/113802 is directed to a device capable of stimulating both back-muscles and abdominal muscles of a wearer for the purpose of reinforcing the entire core, the device may provide sequential or simultaneous stimulation of the back-muscles and abdominal muscles to treat and prevent back pain.

WO 00/41764 is directed to a device for electrotherapeutically stimulating abdominal muscles of a subject comprises a belt which locates a central electrode and a pair of side electrodes on the abdomen of the subject. A signal generator also attached to the belt and connected to the central and side electrodes generates pulsed signals which are applied to the subject through the central and side electrodes for muscle stimulation. The central electrode and the side electrodes are located in the belt so that when the belt is located on the torso with the central electrode over the umbilicus and appropriately stretched around the torso the side electrodes are aligned with respective left and right mid-axillary lines between the rib cage and left and right iliac crests of the subject.

We have gained the following knowledge regarding muscle electrostimulation devices. A plurality of muscles including an abdominal rectus muscle, an oblique abdominal muscle, etc. are located in the abdomen. An abdominal rectus muscle is a muscle running vertically in the front, of the abdomen, and an oblique abdominal muscle is a muscle running vertically on the left and right sides slightly in a slightly oblique manner. In order to train these muscles in a balanced manner, electrostimulation may be supplied to the abdominal rectus muscle and the oblique abdominal muscle. For example, the time required for training will be tripled by training the abdominal rectus muscle, training one oblique abdominal muscle, and then training the other oblique abdominal muscle. If the training time is extended, it is feared that the user may be heavily strained.

Conceivably, three muscle electrostimulation devices may be made available and the three muscles may be trained at the same time using the three muscle electrostimulation devices. However, wearing three muscle electrostimulation devices requires great effort. Moreover, the first device may be detached while the second device or the third device is being fitted to the body. Further, it could be difficult to manipulate the three devices by separate controllers. Based on the foregoing, we have recognized that there is room for improvement in muscle electrostimulation devices in terms of reducing the effort of wearing the device on the body.

In this background, a general purpose of the present disclosure is to provide a muscle electrostimulation device capable of reducing the effort in wearing the device on the body and training an abdominal rectus muscle and an oblique abdominal muscle at the same time.

The invention is defined by the appended claims.

To address the aforementioned issue, a muscle electrostimulation device according to an example of the present disclosure comprises: a main body having a contact surface fitted around an abdomen. The contact surface extends in an X direction encircling the abdomen and in a Y direction perpendicular to the X direction, the main body including: a front part having a front electrode unit adapted to cause an electric current in the X direction in an abdominal rectus muscle; and a pair of extensions extending from respective sides of the front part in the X direction and having side electrode units adapted to cause an electric current in the X direction to flow in an oblique abdominal muscle. Front recesses that recede in the Y direction are provided at positions in side portions of the front part along the Y direction that bisect the front part in the X direction.

Another example of the present disclosure relates to a muscle electrostimulation device. The devices comprises: a main body having a contact surface fitted around an abdomen. The contact surface extends in an X direction encircling the abdomen and in a Y direction perpendicular to the X direction, the main body including: a front part having a front electrode unit adapted to cause an electric current in the X direction in an abdominal rectus muscle; and a pair of extensions extending from respective sides of the front part in the X direction and having side electrode units adapted to cause an electric current in the X direction to flow in an oblique abdominal muscle. A dimension of the pair of extensions in the Y direction is configured to be smaller than a dimension of the front part in the Y direction.

Another example of the present disclosure also relates to a muscle electrostimulation device. The device comprises: a main body having a contact surface fitted around an abdomen. The contact surface extends in an X direction encircling the abdomen, the main body including: a front part having a front electrode unit adapted to cause an electric current in the X direction in an abdominal rectus muscle; and a pair of extensions extending from respective sides of the front part in the X direction and having side electrode units adapted to cause an electric current in the X direction to flow in an oblique abdominal muscle. The main body is provided with a control unit that supplies a voltage to the front electrode unit the pair of side electrode units, and the control unit is configured to switch between an independent control mode for controlling supplied voltages independently and a concurrent control mode for controlling the supplied voltages concurrently.

Optional combinations of the aforementioned constituting elements, and implementations of the invention in the form of methods, apparatuses, and systems may also be practiced as additional modes of the present invention.

Embodiments will now be described, by way of example only, with reference to the accompanying drawings which are meant to be exemplary, not limiting, and wherein like elements are numbered alike in several Figures, in which:
Figs. 1A-1D are schematic diagrams showing a training system employing the muscle electrostimulation device according to the first embodiment;
Fig. 2 is a block diagram showing functional features of the training system employing the muscle electrostimulation device;
Fig. 3 is a front view of the muscle electrostimulation device of Fig. 1;
Fig. 4 is a plan view of the muscle electrostimulation device of Fig. 1;
Figs. 5A and 5B are rear views of the muscle electrostimulation device of Fig. 1;
Fig. 6 is a rear view showing an exemplary arrangement of leads of the muscle electrostimulation device of Fig. 1;
Fig. 7 is a rear view showing an exemplary arrangement of electrode units of the muscle electrostimulation device of Fig. 1;
Fig. 8 is a plan view showing an example of the belt member joined to the muscle electrostimulation device of Fig. 1;
Fig. 9 is a flowchart showing an example of the operation of the muscle electrostimulation device of Fig. 1;
Fig. 10 is a front view of the muscle electrostimulation device according to the second embodiment;
Fig. 11 is a block diagram showing features of the muscle electrostimulation device of Fig. 10; and
Figs. 12A and 12B are side views schematically showing the vicinity of an attachment mechanism of the muscle electrostimulation device of Fig. 10.

The invention will now be described by reference to the preferred embodiments. This does not intend to limit the scope of the present invention, but to exemplify the invention.

The present invention will be described based on preferred embodiments with reference to the drawings. In the embodiments and variations, the same or equivalent constituting elements and members shall be denoted by the same reference numerals, and duplicative explanations will be omitted appropriately. The dimension of members in the drawings shall be enlarged or reduced as appropriate to facilitate understanding. Those of the members that are not material to the description of the embodiments are omitted in the drawings.

Terms including ordinal numbers like first and second are used to describe a series of constituting elements, but those terms are used solely for the purpose of distinguishing one constituting element from another and shall not limit the constituting elements.

### [First embodiment]

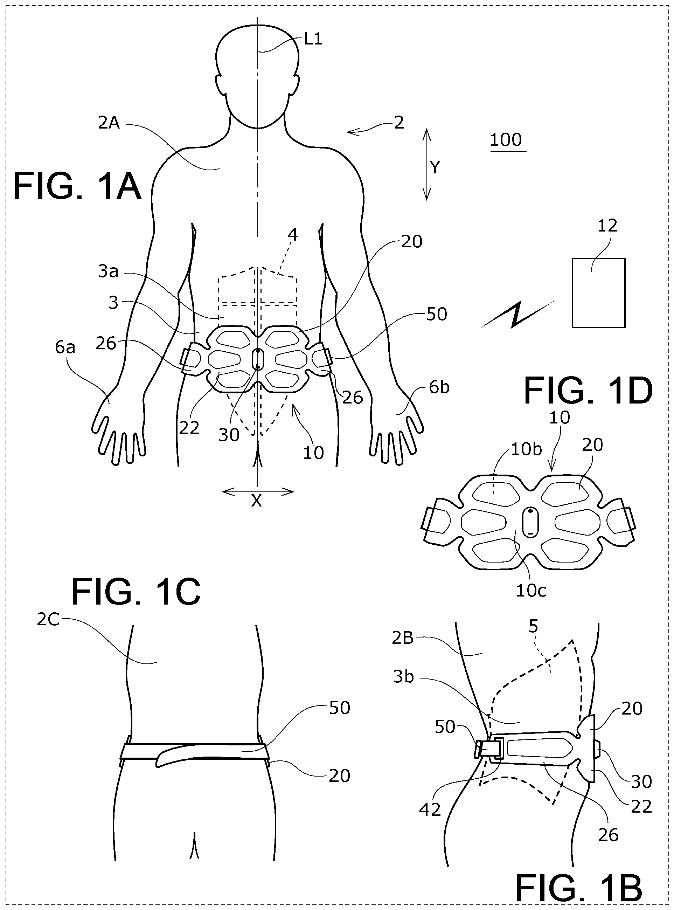

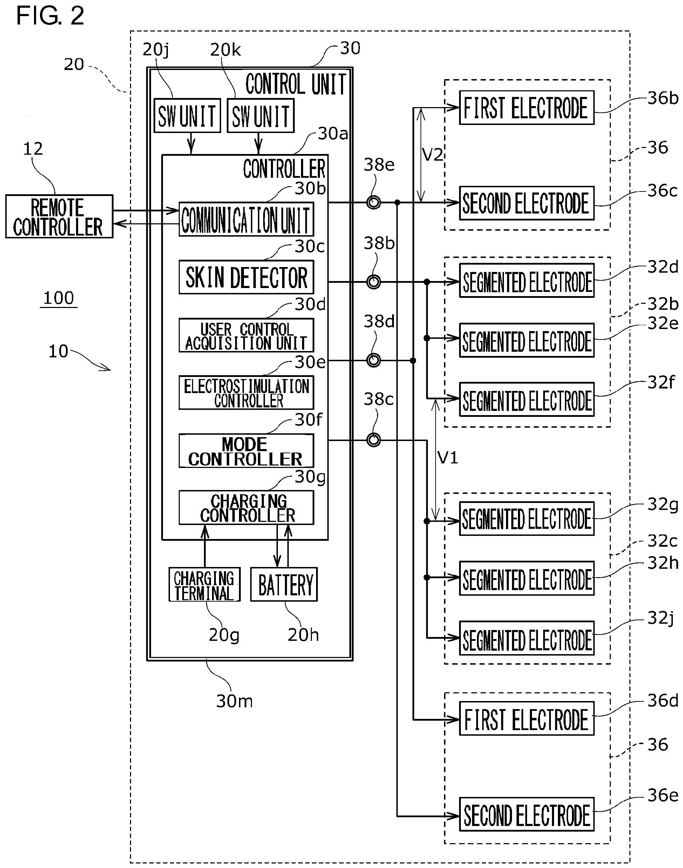

A description will be given of a muscle electrostimulation device 10 with reference to Figs. 1A-9. Figs. 1A-1D are schematic diagrams showing a training system 100 employing the muscle electrostimulation device 10 according to the first embodiment. Fig. 2 is a block diagram showing functional features of the training system 100 employing the muscle electrostimulation device 10. Referring to Figs. 1A-1D, Fig. 1A shows a body 2 viewed from front, Fig. 1B shows the body 2 viewed from left, and Fig. 1C shows the body 2 viewed from back. The training system 100 includes the muscle electrostimulation device 10 and a remote controller 12 . The muscle electrostimulation device 10 according to the first embodiment is fitted to an abdomen 3 of a user. The muscle electrostimulation device 10 supplies electrostimulation to the muscle of the abdomen 3. Further, the muscle electrostimulation device 10 detects an operating condition of the muscle electrostimulation device 10 and outputs a result of detection to the remote controller 12 by wireless or wired connection.

The remote controller 12 is any of a variety of terminals manipulated by the user. For example, the remote controller 12 may be a PC, a tablet, or a smartphone. The remote controller 12 controls the operation of the muscle electrostimulation device 10 in accordance with user manipulation. The remote controller 12 receives the result of detection of the operating condition from the muscle electrostimulation device 10. By way of example, the muscle electrostimulation device 10 may transmit device information to the remote controller 12 by Bluetooth (registered trademark) orWi-Fi (registered trademark) and receive user control information from the remote controller 12.

The remote controller 12 displays information on a touch panel in accordance with the operating condition obtained from the muscle electrostimulation device 10. The user can know the operating condition of the muscle electrostimulation device 10 by viewing the display on the touch panel of the remote controller 12. The remote controller 12 is adapted to detect user control on the touch panel. The user can provide a touch operation by taping the touch panel once lightly with a finger or swipe in a certain direction on the touch panel with a finger pressed against the touch panel. The remote controller 12 is adapted to generate user control information based on the user's touch operation and transmit the information to the muscle electrostimulation device 10.

As shown in Figs. 1A-1D, the muscle electrostimulation device 10 according to the first embodiment is fitted to the abdomen 3 of the body 2 for use. The muscle electrostimulation device 10 has a contact surface 10b fitted around the abdomen 3 when the muscle electrostimulation device 10 is worn. The contact surface 10b extends in the X direction encircling the abdomen 3 and in the Y direction perpendicular to the X direction. The X direction and the Y direction merely define the orientation of the surface of the contact surface 10b. The contact surface 10b may include a portion extending in a direction inclined to the Y direction. The direction perpendicular to the X direction and the Y direction is defined as the Z direction (not shown in Figs. 1A-1D) . The Z direction is perpendicular to the contact surface 10b.

In the example of Figs. 1A-1D, the muscle electrostimulation device 10 is worn such that the Y direction of the contact surface 10b is aligned with the longitudinal direction of the body 2 and the X direction of the contact surface 10b encircles the abdomen 3. It is desired that the muscle electrostimulation device 10 is worn such that the front recesses of the main body 20 described later are aligned with the straight line L1 in the Y direction passing through the navel (not shown) Hereinafter, the Y direction may be denoted as the upward direction or the down direction, and the Z direction may be denoted as the thickness direction. The notation of directions above shall not limit the orientation of the muscle electrostimulation device 10 in use, and the muscle electrostimulation device 10 may be used in any orientation.

The muscle electrostimulation device 10 includes a main body 20 and a belt member 50. The main body 20 is guided around the front of the abdomen 3 and the belt member 50 is guided from one end of the main body 20, guided around the back of the abdomen 3 in the circumferential direction, and is joined to the other end of main body 20. The belt member 50 is an attraction member that attracts the main body 20 toward the abdomen 3 fitted with the muscle electrostimulation device 10. The belt member 50 will be described later.

The main body 20 includes a front part 22, a pair of extensions 26, a control unit 30, and belt joints 42. The front part 22 is fitted to the front of the abdomen 3. The front part 22 is provided with a front electrode unit configured to cause an electric current to flow in an abdominal rectus muscle 4 along the X direction . The pair of extensions 26 extend in respective circumferential directions from the front part 22. The pair of extensions 26 are respectively provided with side electrode units configured to cause an electric current to flow in an oblique abdominal muscle 5 along the circumferential direction. The control unit 30 is an electronic unit for supplying a voltage for muscle stimulation to the front electrode unit and the side electrode units. The belt joints 42 are members for joining two belts of the belt member 50 to the main body 20. The front electrode unit and the side electrode units are sheet electrodes that supply a voltage for muscle stimulation to the portion fitted with the muscle electrostimulation device 10. The sheet electrode can be formed by printing an electroconductive material such as silver paste or by etching.

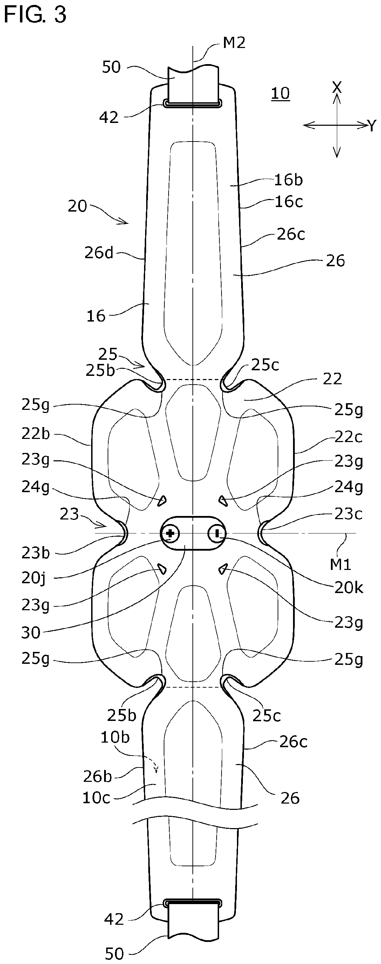

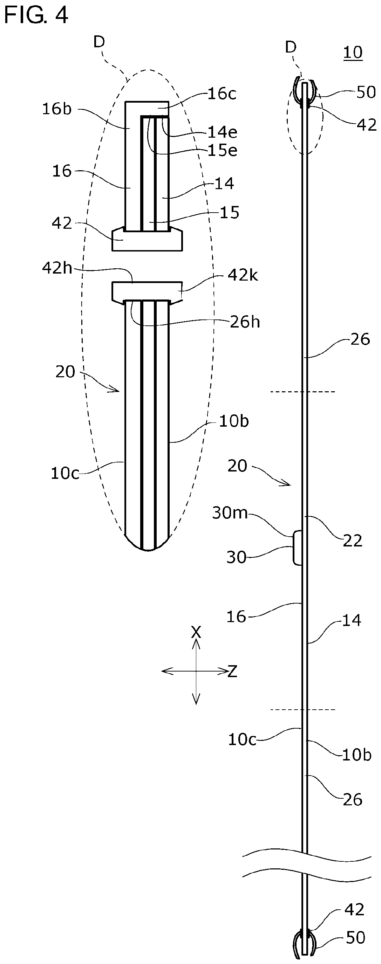

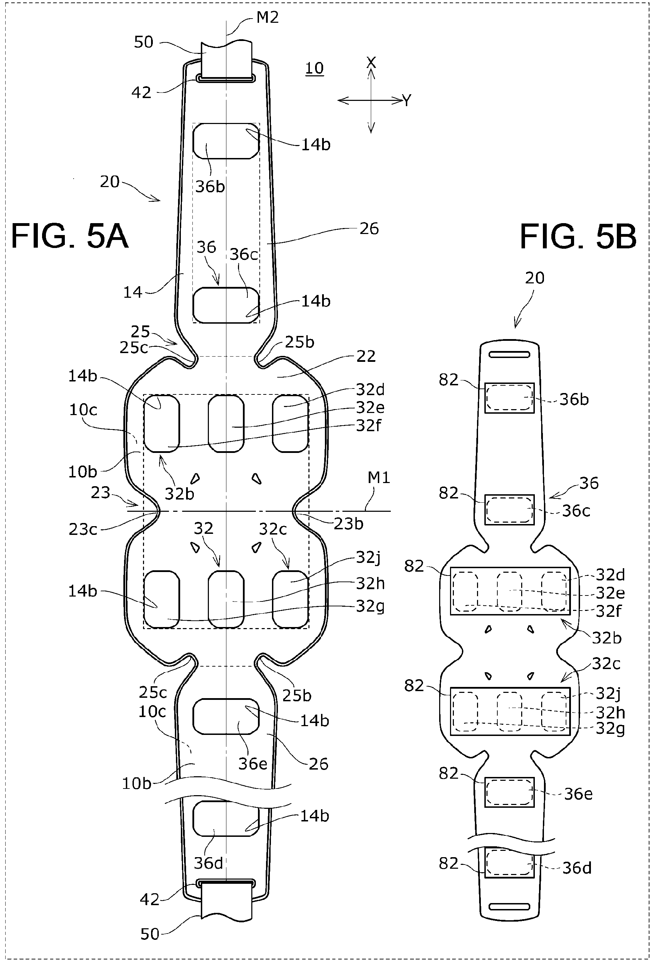

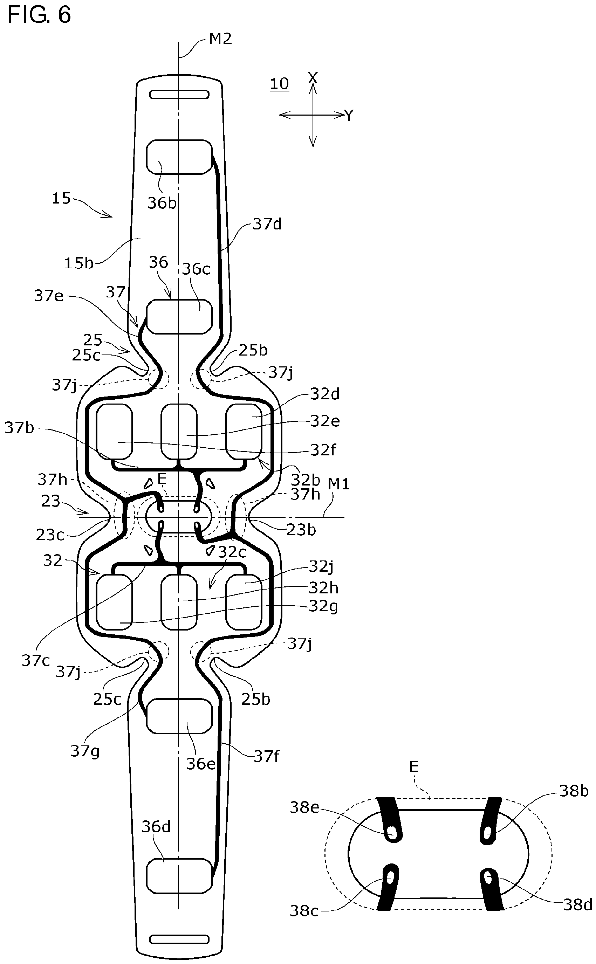

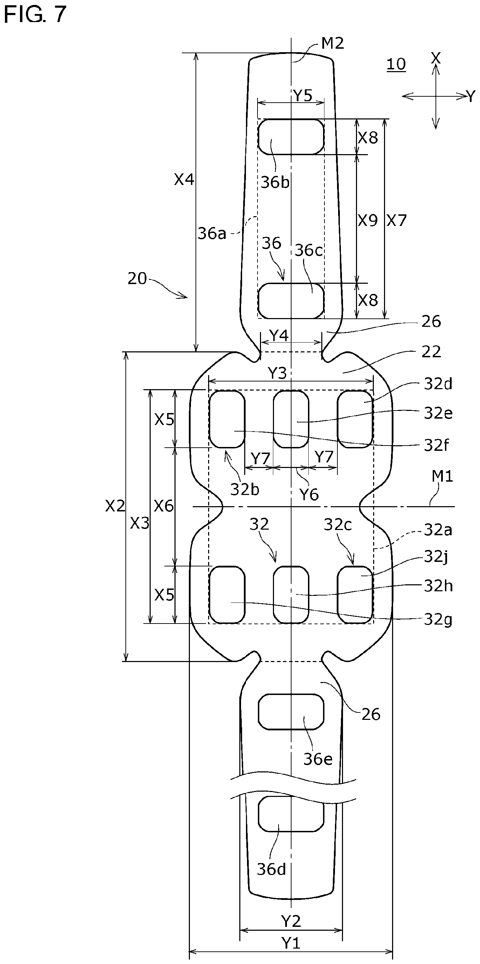

Fig. 3 is a front view of the muscle electrostimulation device 10. Fig. 4 is a plan view of the muscle electrostimulation device 10. Figs. 5A and 5B are rear views of the muscle electrostimulation device 10. Fig. 6 is a rear view showing an exemplary arrangement of leads 37 of the muscle electrostimulation device 10; Fig. 7 is a rear view showing an exemplary arrangement of electrode units of the muscle electrostimulation device 10; Figs. 3-7 are front views of the contact surface 10b of the respective units by expanding the muscle electrostimulation device 10. As shown in Figs. 3-7, the X direction, which corresponds to the circumferential direction of the body 2, and the Y direction, which corresponds to the vertical direction of the body 2, are indicated by straight lines of arrows X and Y.

### (Main body)

The main body 20 may include a layered structure. By way of example, the main body 20 includes a first base member 14, a second base member 15, and a cover member 16. The first base member 14 is a member fitted to the body 2 and exhibits a flat sheet shape when expanded. A contact surface 10b in contact with the abdomen 3 is provided on the back surface of the first base member 14. The first base member 14 has an opening 14b that exposes the front electrode unit 32 and the side electrode units 36 on the contact surface 10b. The first base member 14 may be configured by combining a plurality of members. The first base member 14 of this example is configured such that the portion corresponding to the front part 22 and the portion corresponding to the pair of extensions 26 are integrated. The durability of the leads 37 of the muscle electrostimulation device 10 of this example is ensured to be higher than when the first base member 14 is not provided.

The second base member 15 is a member supporting the front electrode unit 32, the side electrode units 36, and the leads 37 and exhibits a flat sheet shape when expanded. The second base member 15 may be configured by combining a plurality of members. The second base member 15 of this example is configured such that the portion corresponding to the front part 22 and the portion corresponding to the pair of extensions 26 are integrated. The rear surface of the second base member 15 is provided with the front electrode unit 32 and the side electrode units 36. The front electrode unit 32 and the side electrode units 36 can be formed by printing an electroconductive material such as silver paste. The front electrode unit 32 and the side electrode units 36 may be formed by etching. The second base member 15 is provided with the leads 37 electrically connected to the front electrode unit 32 and the side electrode units 36. For example, the leads 37 can be formed by printing an electroconductive material such as silver paste. The leads 37 may be formed by etching. The leads 37 may be referred to as a wiring pattern.

The cover member 16 is a member built on the side of the second base member 15 opposite to the first base member 14. The cover member 16 includes a sheet portion 16b exhibiting a flat sheet shape when expanded, and an edge cover portion 16c extending from the outer edge of the sheet portion 16b toward the second base member 15 in the thickness direction (Z direction) . An outer surface portion 10c of the sheet portion 16b of the cover member 16 is provided with the control unit 30. The cover member 16 may be configured by combining a plurality of members. The cover member 16 of this example is configured to cover the front part 22 and the pair of extensions 26 so as to integrate them.

By forming the first base member 14 and the second base member 15 to be thin, outer edges 14e and 15e thereof will also be thin. When the muscle electrostimulation device 10 is worn on the body 2, the outer edges 14e, 15e may be in contact with the body, causing the user to feel uncomfortable. To address this, the cover member 16 of this example has the edge cover portion 16c that covers the outer edges 14e, 15e of the first base member 14 and the second base member 15. Since the edge cover portion 16c covers the outer edges 14e, 15e, the area of contact between the main body 20 and the body 2 is increased so that the user can feel more comfortable than otherwise. The edge cover portion 16c of this example is formed in the shape of a band encircling the outer edges 14e, 15e. The rigidity of the cover member 16 of this example is ensured to be higher than when the edge cover portion 16c is not provided. Additionally, when the muscle electrostimulation device 10 is worn on the body 2, the outer edges 14e, 15e may bite into the skin. By providing the edge cover portion 16c, the stimulus and pain on the skin can be mitigated.

The embodiment is non-limiting as to the material of the first base member 14 and the second base member 15 . Any insulative material that is flexible enough to be wrapped around the body 2 may serve the purpose. The first base member 14 and the second base member 15 is made of an elastomer such as polyethylene terephthalate-based resin. The embodiment is non-limiting as to the material of the cover member 16. Any insulative material that is flexible enough to be wrapped around the body 2 may serve the purpose. By way of example, the cover member 16 may be made of a resin material harder than the first base member 14 and the second base member 15. The cover member 16 of this example is made of an elastomer such as silicon resin. In this case, the cover member 16 protects the first base member 14 and the second base member 15, inhibits damage from handling, and is capable of imparting the main body 20 with suitable rigidity. The cover member 16 made of silicon resin is adapted to cover the front through the sides of the abdomen 3. By using the muscle electrostimulation device 10, the body temperature is transmitted to the silicon resin and stored therein. Therefore, the user can feel the warmth while using the device, and it is expected that the blood flow is improved due to the hyperthermic effect.

### (front part)

The front part 22 is fitted to a position corresponding to the abdominal rectus muscle 4 at the front of the abdomen 3. The front part 22 is provided with the front electrode unit 32 configured to cause an electric current in the abdominal rectus muscle 4 along the circumferential direction. The front part 22 extends in the X direction and in the Y direction and is formed to be thin in the Z direction. The front part 22 has a shape that can substantially be considered as a rectangle having a dimension Y1 in the Y direction and a dimension X2 in the X direction in a front view. Hereinafter, the shape that can substantially be considered as a rectangle includes a shape provided with a pair of opposite sides having a length of 30% or larger of the entire length. The longitudinal direction of the front part 22 is arranged along the X direction.

The space between the muscle electrostimulation device 10 and the abdomen 3 may become humid due to the sweat during the training. To address this, the front part 22 of this example is provided with a plurality of (e.g., four) air vent holes 23g.

As shown in Fig. 3, the air vent holes 23g are provided at positions distanced from the electrodes and the leads and surrounding the control unit 30. In this case, the humidity between the muscle electrostimulation device 10 and the abdomen 3 is mitigated as compared with a configuration in which air vent holes are not provided. An additional benefit of providing the air vent holes 23g is that the flexibility of the front part 22 is improved. By modifying the number and arrangement of air vent holes 23g, the flexibility of the front part 22 can be adjusted.

By way of example, the front part 22 is formed to be symmetrical with respect to the control unit 30 in the X direction and in the Y direction.

### (Front recesses)

For ease of wearing, it is desired that the central position of the front part 22 of the muscle electrostimulation device 10 in the X direction be easily identified. To address this, the muscle electrostimulation device 10 of this example is provided with front recesses 23 at the central position of the front part 22. The front recesses 23 are provided at positions in side portions 22b, 22c of the front part 22 along the Y direction that bisect the front part 22 in the X direction. Fig. 3 shows a bisector M1 that bisects the front part 22 in the X direction and a bisector M2 that bisects the front part 22 in the Y direction. The front recesses 23 recede in the Y direction . The front recesses 23 of this example include a first recess 23b and a second recess 23c provided in side portions 22b, 22c on the respective sides in the Y direction.

It is desired that the central position of the front recesses 23 be identifiable by a touch at the fingertip as well as visually. To meet this requirement, the front recesses 23 of this example have an opening that opens in the X direction with a size enough to rest the fingertip. If the front recesses 23 are too shallow, the identifiability of the central position is lowered. In this respect, the depth of the front recesses 23 of this example may be configured with a size capable of accepting 1/4 of the distal segment of the fingertip.

### (Boundary recesses)

In many cases, the circumferential surface of the abdomen 3 includes curved surfaces that undulate in a complicated manner. For this reason, if the main body 20 is fitted to the abdomen 3, the extensions 26 are fitted to the abdomen 3 such that they are twisted with respect to the front part 22. If the extensions 26 is difficult to twist, the twisting moment created between the front part 22 and the extensions 26 grows so that the user feels less comfortable wearing the device on the abdomen 3. To address this, boundary recesses 25 that recede inward in the Y direction are provided on respective sides of the main body 20 along the Y direction at boundaries between the front part 22 and the pair of extensions 26. This makes it easier for the extensions 26 to be twisted and allows the user to feel more comfortable wearing the device on the abdomen 3 than when the boundary recesses 25 are not provided.

The boundary recesses 25 of this example include a first boundary recess 25b and a second boundary recess 25c provided in side portions 26b, 26c on respective sides in the Y direction. This makes it easier for the extensions 26 to be twisted and allows the user to feel more comfortable wearing the device on the abdomen 3 than when one of the boundary recesses is not provided. The boundary recesses 25 are shaped to approach the bisector M1 toward the far end. In essence, the boundary recesses 25 are formed to be inclined with respect to the X direction. Unlike the case where the inclination is not provided, the area subject to deformation due to the twist is enlarged in the X direction so that the stress from the twist can be distributed in the X direction. As shown in Fig. 7, the dimension Y4, in the Y direction, between the portions of the first boundary recess 25b and the second boundary recess 25c closest to each other in the Y direction is larger than the dimension Y5 described later and smaller than the dimension Y2 described later.

### (Leads)

As shown in Fig. 6, the leads 37 of this example are provided on a rear surface 15b of the second base member 15. The leads 37 include a wiring 37b, a wiring 37c, a wiring 37d, a wiring 37e, a wiring 37f, a wiring 37g, curved portions 37h, and curved portions 37j. The wiring 37b electrically connects a terminal 38b and an electrode group 32b. The wiring 37c electrically connects a terminal 38c and an electrode group 32c. The wiring 37d electrically connects a terminal 38d and a first electrode 36b. The wiring 37e electrically connects a terminal 38e and a second electrode 36c. The wiring 37f electrically connects the terminal 38d and a first electrode 36d. The wiring 37g electrically connects the terminal 38e and a second electrode 36e. The curved portions 37h are provided to conform to the front recesses 23. The curved portions 37j are provided to conform to the boundary recesses 25.

In the presence of the front recesses 23, the leads 37 may be too close to the outer edge of the second base member 15 around the front recesses 23. To address this, the leads 37 of this example have curved portions 37h curved inward in the Y direction to conform to the front recesses 23. By providing the curved portions 37h, the front recesses 23 can be shaped as desired.

In the presence of the front recesses 23, the stress may be concentrated in the portion of the main body 20 where the front recesses 23 are provided, which may result in a trouble such as a crack in that portion. To address this, the portion of the main body 20 of this example provided with the front recesses 23 is provided with a thick portion 24g formed to have a larger thickness than elsewhere. The shape of the thick portion 24g may be defined by a simulation in accordance with the desired durability.

In the presence of the boundary recesses 25, the leads 37 may be too close to the outer edge of the second base member 15 around the boundary recesses 25. To address this, the leads 37 of this example have curved portions 37j curved inward in the Y direction to conform to the boundary recesses 25. By providing the curved portions 37j, the boundary recesses 25 can be shaped as desired.

In the presence of the boundary recesses 25, the stress may be concentrated in the portion of the main body 20 where the boundary recesses 25 are provided, which may result in a trouble such as a crack in that portion. To address this, the portion of the main body 20 of this example provided with the boundary recesses 25 is provided with a thick portion 25g formed to have a larger thickness than elsewhere. The shape of the thick portion 25g may be defined by a simulation in accordance with the desired durability. In this example, the thick portion 25g is shaped such that the cross section thereof along the Z direction rises from the edge of the corresponding one of the boundary recesses 25 to form an apex, and the thick portion 25g grows less thick away from the apex and toward the center.

### (Front electrode unit)

As shown in Fig. 7, the front electrode unit 32 is provided in a placement area 32a having a dimension X3 in the X direction and a dimension Y3 in the Y direction. The front electrode unit 32 may include a plurality of electrodes arranged at a distance X6 in the X direction. The distance X6 may be defined to conform to the width of the abdominal rectus muscle 4. The front electrode unit 32 of this example includes a pair of electrode groups 32b, 32c arranged at a distance in the X direction, which corresponds to the circumferential direction of the abdomen 3. The electrode groups 32b, 32c are arranged at a distance X6 in the X direction. Specifically, the electrode groups 32b, 32c are spaced apart in the X direction.

The electrode group 32b includes a plurality of (e.g., three) segmented electrodes 32d, 32e, 32f arranged at equal spacing in the Y direction. The electrode group 32c includes a plurality of (e.g., three) segmented electrodes 32g, 32h, 32j arranged at equal spacing in the Y direction. By way of example, a dimension X5 of the segmented electrodes of the front electrode unit 32 of this example in the X direction is larger than a dimension Y6 in the Y direction, and the dimension Y6 is larger than a distance Y7 between the segmented electrodes. By way of example, the electrodes of the front electrode unit 32 have a shape substantially considered as a rectangle. When the muscle electrostimulation device 10 is fitted to the abdomen 3, the pair of electrode groups 32b, 32c are arranged in areas aligned with the abdominal rectus muscle 4 and cause a circumferential electric current to flow in the abdominal rectus muscle 4. In this example, the segmented electrodes of the pair of electrode groups 32b, 32c are arranged in alignment with sections partitioned by the intersectio tendinea of the abdominal rectus muscle 4.

By way of example, the front electrode unit 32 is arranged in symmetry with respect to the control unit 30 in the X direction and in the Y direction. Specifically, the front electrode unit 32 is arranged in line symmetry with respect to a straight line bisecting the placement area 32a of the front electrode unit 32 in the X direction. The front electrode unit 32 may be placed in line symmetry with respect to the bisector M1. Specifically, the front electrode unit 32 of this example is placed in line symmetry with respect to a straight line bisecting the placement area 32a of the front electrode unit 32 in the Y direction. The front electrode unit 32 may be placed in line symmetry with respect to the bisector M2.

When the muscle electrostimulation device 10 is fitted to the abdomen 3 for use, a gel pad 82 (see Figs. 5A and 5B) is placed between the front electrode unit 32 and the abdomen 3. The gel pad 82 is sticky. The gel pad 82 plays the role of securing conductivity between the body and each electrode with its stickiness. The gel pad 82 is pasted to each electrode unit of the muscle electrostimulation device 10. The muscle electrostimulation device 10 is fitted to the abdomen 3 with the gel pad 82 pasted. The muscle electrostimulation device 10 is supported on the abdomen 3 by the stickiness of the gel pad 82.

The gel pad 82 may be pasted one each to the segmented electrodes of the front electrode unit 32. In this example, the gel pad 82 is shaped to cover a plurality of segmented electrodes collectively, as shown in Figs. 5A and 5B. In essence, the gel pad 82 is configured to be pasted one each to the electrode groups 32b, 32c. This reduces the effort required to attach the gel pad 82 as compared with a case of providing one pad in each segmented electrode.

### (Side electrode units)

As shown in Fig. 7, each of the side electrode units 36 is arranged in a placement area 36a having a dimension X7 in the X direction and a dimension Y5 in the Y direction. The side electrode units 36 may include a plurality of electrodes arranged at a distance X9 in the X direction. The distance X9 may be defined to conform to the width of the oblique abdominal muscle 5. The side electrode units 36 of this example include first electrodes 36b, 36d and second electrodes 36c, 36e spaced apart in the X direction. Specifically, the first electrodes 36b, 36d and the second electrodes 36c, 36e are spaced apart in the X direction, which corresponds to the circumferential direction of the abdomen 3. The term "the side electrodes" is used to refer to the first electrodes 36b, 36d and the second electrodes 36c, 36e collectively. The first electrode 36b and the second electrode 36c are arranged in one of the extensions 26, spaced apart by the distance X9 in the X direction. When the muscle electrostimulation device 10 is fitted to the abdomen 3, the first electrode 36b and the second electrode 36c are arranged around the oblique abdominal muscle 5 in the X direction, which corresponds to circumferential direction, and cause an electric current to flow in the X direction, which corresponds to the circumferential direction, in the oblique abdominal muscle 5.

The first electrode 36d and the second electrode 36e are arranged in the other of the extensions 26, spaced apart in the X direction. When the muscle electrostimulation device 10 is fitted to the abdomen 3, the first electrode 36d and the second electrode 36e are arranged around the oblique abdominal muscle 5 in the X direction, which corresponds to circumferential direction, and cause an electric current to flow in the X direction, which corresponds to the circumferential direction, in the oblique abdominal muscle 5. By way of example, a dimension Y5 of the side electrodes of the side electrode units 36 of this example in the Y direction is larger than a dimension X8 in the X direction.

By way of example, each of the side electrodes of the side electrode units 36 has a shape substantially considered as a rectangle. The shape and arrangement of the electrode units may be defined in accordance with the curved surface of the body to conform to the curved surface. For example, if the abdominal circumference is large, the curved surface of the abdominal rectus muscle 4 overhangs outward and is twisted in the shape of a bow. Therefore, the shape and arrangement of the electrode units may be defined to conform to the curved surface overhanging in the shape of a bow.

By way of example, the side electrode units 36 are arranged in symmetry with respect to the control unit 30 in the X direction and in the Y direction. Specifically, the side electrode units 36 are arranged in line symmetry with respect to a straight line bisecting the placement area 32a of the front electrode unit 32 in the X direction. The side electrode units 36 may be arranged in line symmetry with respect to the bisector M1. Specifically, the side electrode units 36 of this example are arranged in line symmetry with respect to a straight line bisecting the placement area 32a of the front electrode unit 32 n the Y direction. The side electrode units 36 may be arranged in line symmetry with respect to the bisector M2.

The gel pad 82 is placed between the side electrode units 36 and the abdomen 3 when the muscle electrostimulation device 10 is fitted to the abdomen 3. By way of example, as shown in Figs. 5A and 5B, the gel pad 82 of a shape that covers each one of the side electrodes is pasted to each one of the side electrodes for use.

### (Extensions)

The pair of extensions 26 are fitted at positions aligned with the oblique abdominal muscle 5 at the sides of the abdomen 3. The extensions 26 are respectively provided with the side electrode units 36 that cause an electric current to flow in the oblique abdominal muscle 5. The extensions 26 extend in the X direction and in the Y direction and are formed to be thin in the Z direction. The extensions 26 have a shape substantially considered as a rectangle in which the longitudinal direction is aligned with the X direction.

By way of example, the dimension Y2 of the extensions 26 of this example in the Y direction is smaller than the dimension Y1 of the front part 22 in the Y direction. The boundary between the front part 22 and the extensions 26 may be formed as a step. By way of example, the extensions 26 of this example have a shape that can be substantially considered as a rectangle, and a dimension X4 in the X direction is larger than the dimension Y2 in the Y direction. By way of example, the dimension Y2 of the pair of extensions 26 of this example in the Y direction is smaller than the dimension Y3 of the placement area 32a of the front electrode unit in the Y direction. One of the pair of extensions 26 is provided with the corresponding one of the side electrode units 36 having the first electrode 36b and the second electrode 36c arranged at a distance in the X direction. By way of example, the dimension Y2 of the pair of extensions of this example in the Y direction is smaller than the distance X9 between the first electrode 36b and the second electrode 36c in the X direction. In the example of Fig. 7, the dimensions and distances in the Y direction are related such that distance Y7<dimension Y6<dimension Y4<dimensionY5<dimension Y2<dimension Y1. In the example of Fig. 7, the dimensions and distances in the X direction are related such that dimension X8<dimension X5<distance X6<distance X9<dimension X7<dimension X3<dimension X4<dimension X2.

The abdominal circumference varies from person to person. It is therefore considered that the distance from the front of the abdomen 3 to the oblique abdominal muscle 5 varies significantly from person to person. For this reason, too small a distance X9 between the first electrode 36b and the second electrode 36c may narrow the range in the oblique abdominal muscle 5 in which an electric current flows. To address this in this example, the distance X9 between the first electrode 36b and the second electrode 36c is defined to be larger than the distance X6 between the electrode group 32b and the electrode group 32c. In this case, the range in the oblique abdominal muscle 5 in which an electric current flows is larger than when the distance X9 is smaller than the distance X6.

The abdominal circumference varies from person to person. It is therefore considered that the range in which the direction perpendicular to the direction of extension of the muscle varies significantly from person to person. To address this, the electrodes of the muscle electrostimulation device 10 are arranged to cause a circumferential electric current to flow in the abdominal rectus muscle 4 and the oblique abdominal muscle 5. In essence, the electrodes are adapted to cause an electric current to flow in a direction substantially perpendicular to the direction extension of these muscles. In this case, it is expected that a wider range of muscle is stimulated than when an electric current is caused to flow in the direction of extension of the muscle.

Since the electrodes are arranged at a distance in a direction substantially perpendicular to the direction of twist of the extensions 26, it is ensured that the electrodes are in contact with the body in more stable manner than when the electrodes are placed in alignment with the direction of twist.

The direction of an electric current flowing in the abdominal rectus muscle 4 and the direction of an electric current flowing in the oblique abdominal muscle 5 can be defined according to the polarity of the voltage applied to the front electrode unit 32 and the side electrode units 36. The direction of an electric current flowing in the abdominal rectus muscle 4 may be identical to or opposite to the direction of an electric current flowing in the oblique abdominal muscle 5. In this example, the polarity of the voltage applied to the front electrode unit 32 and the side electrode units 36 is defined so that an electric current flows in the abdominal rectus muscle 4 in a direction opposite to an electric current flowing in the oblique abdominal muscle 5.

### (Control unit)

A description will now be given of the control unit 30. The blocks of the control unit 30 shown in Fig. 2 and those of the control unit 70 described later can be implemented in hardware such as devices or mechanical components exemplified by a Central Processing Unit (CPU) of a computer, and in software such as a computer program etc. Fig. 2 depicts functional blocks implemented by the cooperation of these elements. Therefore, it will be understood by those skilled in the art that the functional blocks may be implemented in a variety of manners by a combination of hardware and software.

The control unit 30 includes a controller 30a, switch units 20j, 20k, a charging terminal 20g, and a battery 20h, which are housed in a housing 30m. The housing 30m is provided at the center of the main body 20 and is configured as an outer shell of the control unit 30. The housing 30m can be made of various resin materials. The switch units 20j, 2k are provided on the front of the control unit 30. The switch units may be denoted as SW units. The switch units 20j, 20k detect that the user holds down the switch and output a detection result to the controller30a. The switch units 20j, 20k may include an electrical contact that is closed while the switch is not being held down and is opened when the switch is held down. The surface of the switch unit 20j of this example is labeled by a plus sign, and the surface of the switch unit 20k is labeled by a minus sign.

The battery 20h is electrically connected to the controller 30a and supplies electric power to the controller 30a. The battery 20h may be a primary battery. In this example, a rechargeable lithium ion battery is employed. The battery 20h may be replaceable, but a built-in battery is employed in this example. The charging terminal 20g receives electric power to charge the battery 20h and outputs the power to the controller 30a. Various connectors can be used for the charging terminal 20g. In this example, a USB (registered trademark) connector is employed. For example, the charging terminal 20g is provided on the bottom surface of the control unit 30. The battery 20h is charged by the electric power received by the charging terminal 20g. The battery 20h may be configured to be charged by a charging system of non-contact type such as a wireless charging system.

The controller 30a includes a communication unit 30b, a skin detector 30c, a user control acquisition unit 30d, an electrostimulation controller 30e, a charging controller 30g, and a mode controller 30f. The communication unit 30b is adapted to transmit an operating condition of the muscle electrostimulation device 10 to the remote controller 12. The remote controller 12 displays the operating condition acquired from the communication unit 30b. The communication unit 30b receives user control information based on a touch operation input in the remote controller 12. The controller 30a controls the operation of the muscle electrostimulation device 10 in accordance with the user control information acquired via the communication unit 30b.

The user control acquisition unit 30d acquires a detection result from the switch units 20j , 20k. The user control acquisition unit 30d detects the voltage commensurate with the resistance of the switch. If the detected voltage is equal to higher than a threshold value, the user control acquisition unit 30d determines that the switch unit is held down. If the detected voltage is less than the threshold value, the user control acquisition unit 30d determines that the switch unit is not held down. The controller 30a determines a method of controlling the muscle electrostimulation device 10 in accordance with a combination of statuses of the switch units indicating whether the switch is held down and with a duration of the switch unit being held down.

User control through the switch units 20j, 20k may be prohibited while the remote controller 12 is being manipulated. The muscle electrostimulation device 10 of this example is configured such that user control through the switch units 20j, 20k is enabled even when the remote controller 12 is being manipulated. In this case, the user can manipulate the muscle electrostimulation device 10 with the switch units 20j, 20k even when the user is away from the remote controller 12 and cannot use the remote controller 12 for manipulation.

The skin detector 30c detects whether the electrodes are in contact with the skin. The skin detector 30c detects the resistance between the electrode group 32b and the electrode group 32c. The skin detector 30c determines that the electrodes are in contact with the skin when the detected resistance is less than a threshold value and determines that the electrodes are not in contact with the skin when the detected resistance value is equal to or higher than the threshold value.

When the skin detector 30c detects that the electrodes are in contact with the skin, the electrostimulation controller 30e supplies electric power commensurate with a preset output voltage for a predetermined duration of operation (e.g., 20 minutes) and at a predetermined interval. In essence, the abdomen 3 of the user is electrically stimulated for the duration of operation. The output voltage can be altered by manipulating the remote controller 12 or the switch units 20j , 20k. The output voltage in the electrostimulation controller 30e of this example rises each time the switch unit 20j is held down and drops each time the switch unit 20k is held down.

The charging controller 30g controls the electric power for charging received in the charging terminal 20g and supplies the electric power to the battery 20h. The charging controller 30g controls the magnitude of the electric current supplied to the battery 20h in accordance with the charging rate of the battery 20h. For example, if the charging rate is low, the charging controller 30g increases the electric current supplied to the battery 20h. If the charging rate is high, the charging controller 30g decreases the electric current supplied to the battery 20h. If a margin of increase in the charging rate is small in relation to the amount of charge stored, it is determined that battery has failed and the battery is not charged further.

### (Mode controller)

The control unit 30 is adapted to control a first voltage V1 supplied to the front electrode unit 32 and a second voltage V2 supplied to the pair of side electrode units 36. The control unit 30 has an independent control mode for controlling the first voltage V1 and the second voltage V2 independently and a concurrent control mode for controlling the first voltage V1 and the second voltage V2 concurrently.

In the independent control mode, one of the first voltage V1 and the second voltage V2 may be selected and controlled if it is desired that stimulation to one of the abdominal rectus muscle 4 and the oblique abdominal muscle 5 be regulated. For example, one of the first voltage V1 and the second voltage V2 can be fixed, while the other is increased or decreased in the independent control mode. In the independent mode, one of the first voltage V1 and the second voltage V2 can be maintained at zero, while the other is increased or decreased.

In the concurrent control mode, the first voltage V1 and the second voltage V2 are controlled in coordination when it is desired to regulate the stimulation to the abdominal rectus muscle 4 and the oblique abdominal muscle 5 in coordination. For example, the first voltage V1 and the second voltage V2 can be increased or decreased concurrently in the concurrent control mode. In the concurrent control mode, the first voltage V1 and the second voltage V2 can be increased or decreased concurrently such that the voltages are equal to each other. In the concurrent control mode, the first voltage V1 and the second voltage V2 are increased or decreased concurrently such that a voltage difference is maintained.

The mode controller 30f exercises control to switch between the independent control mode and the concurrent control mode. The electrostimulation controller 30e is adapted to switch between the independent control mode and the concurrent control mode by manipulating the remote controller 12 or the switch units 20j, 20k. When the mode is switched to the concurrent control mode, the first voltage V1 and the second voltage V2 are regulated in coordination. When the mode is switched to the independent control mode, one of the first voltage V1 and the second voltage V2 is fixed, while the other is increased or decreased.

### (Belt joints)

The pair of extensions 26 may be provided with a joint for attachment of a member for securing the main body 20 looped around the abdomen 3. In this example, a pair of belt joints 42 for joining the belts of the belt member 50 for attaching the main body 20 looped around the abdomen 3 to the abdomen 3. Structures based on different principles may be employed for the belt joints. The belt joints 42 of this example include rectangular tube members 42k encircling a hole 42h through which the belt member 50 is run. The rectangular tube members 42k present a shape of a substantial rectangle extending longitudinally in the Y direction in a front view. A substantially rectangular hole 42h elongated in the Y direction is provided at the center of the rectangular tube members 42k. The rectangular tube members 42k may be made of a resin material by way of an example. The belt joints 42 may be made of a resin material by way of an example. The pair of rectangular tube members 42k are set and fitted in attachment holes 26h formed at distal ends of extensions 26 away from the front part 22 in the X direction.

### (Belt member)

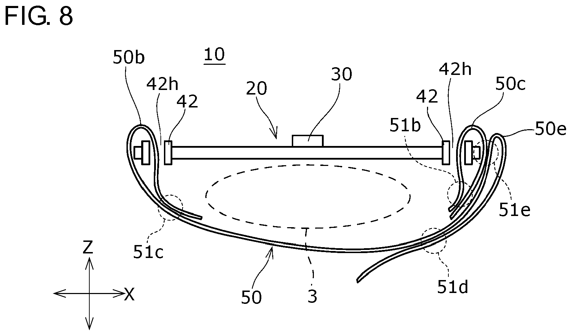

Fig. 8 is a plan view showing an example of the belt member 50 joined to the muscle electrostimulation device 10. The belt member 50 bridges one of the pair of belt joints 42 and the other of the pair of belt joints 42 via the back of the abdomen 3. A belt member configured as a cord, a band, etc. may be employed. The belt member 50 of this example is a band-like member.

The belt member 50 includes a first belt member 50b and a second belt member 50c. The second belt member 50c is run through the hole 42h of one of the belt joints 42 and then folded back. The ends of the second belt member 50c are joined together by a hook-and-loop fastener 51b. The second belt member 50c may be formed to be shorter than the first belt member 50b.

One end of the first belt member 50b is run through the hole 42h of the other of the belt joints 42 and folded back. The end that is folded back is joined to an intermediate portion of the first belt member 50b by a hook-and-loop fastener 51c. The other end of the first belt member 50b is folded back for adjustment of the length so as form a folded-back portion 50e. The folded-back portion 50e is joined to another intermediate portion of the first belt member 50b by a hook-and-loop fastener 51d.

The folded-back portion 50e is adapted to be joined to the second belt member 50c by a hook-and-loop fastener 51e. The muscle electrostimulation device 10 is attached around the abdomen 3 of the user while the folded-back portion 50e is detached from the second belt member 50c. The muscle electrostimulation device 10 is fitted to the abdomen 3 by joining the folded-back portion 50e to the second belt member 50c while the muscle electrostimulation device 10 is fitted around the circumference of the abdomen 3. By altering the position where the folded-back portion 50e is folded back, the circumferential length can be easily adjusted.

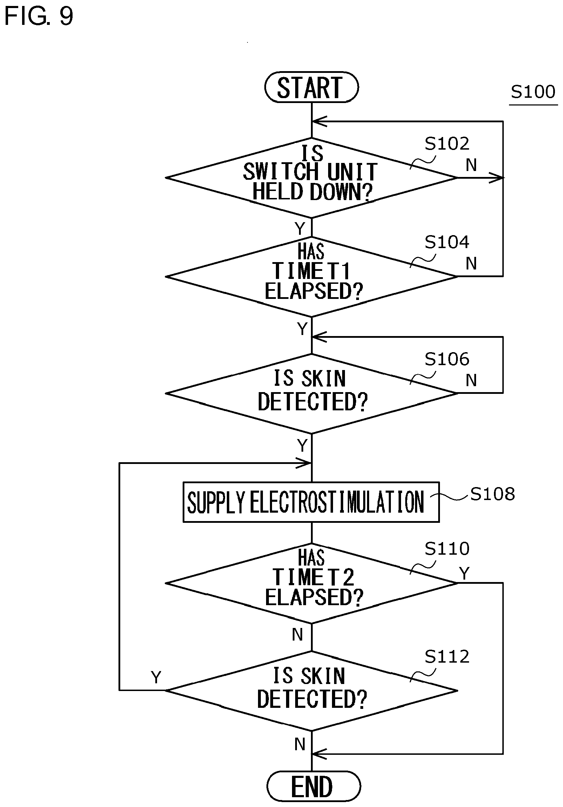

A description will be given of the operation of the muscle electrostimulation device 10 configured as described above. Fig. 9 is a flowchart showing an example of the operation of the muscle electrostimulation device 10. Fig. 9 shows a process S100 performed since power is turned on while the user is wearing the muscle electrostimulation device 10 on the abdomen 3 to start electrostimulation to the abdomen 3 until the electrostimulation is terminated.

When the process is started, the controller 30a determines whether the switch unit 20j is held down (step S102). If the switch unit 20j is not held down (N in step S102), the controller 30a returns control to step S102 and repeats step S102. If the switch unit 20j is being held down (Y in S102), the controller 30a determines whether a predetermined period of time T1 has elapsed (step S104).

If the period of time T1 has not elapsed (N in step S104), the controller 30a returns control to step S102 and repeats steps S102-S104. If the period of time has elapsed (Y in step S104), the controller 30a shifts to step S106. The period of time T1 in step S104 can be defined in accordance with a desired specification. The period of time T1 is defined to be 2 seconds in this example. In essence, the user can turn on the muscle electrostimulation device 10 by holding down the switch unit 20j for 2 seconds.

In step S106, the controller 30a determines whether the electrodes are in contact with the skin (step S106). If the electrodes are not in contact with the skin (N in S106), the controller 30a returns the process to step S106 after waiting a predetermined period of time.

If the electrodes are in contact with the skin (Y in S106), the controller 30a starts supplying electrostimulation to the abdomen 3 (step S108). In this step, the electrostimulation controller 30e supplies electric power that varies at a predetermined interval to at least one of the front electrode unit 32 and the side electrode units 36.

The controller 30a having performed step S108 determines whether a predetermined period of time T2 has elapsed (step S110) . The period of time T2 in step S110 can be defined in accordance with a desired training time. The period of time T2 is defined to be 20 minutes in this example. In essence, the user can continue training until 20 minutes elapses. If the period of time T2 has not elapsed (N in S110), the controller 30a determines whether the electrodes are in contact with the skin (step S112).

If the electrodes are in contact with the skin (Y in S112), the controller 30a returns the process to step S108 and continues to supply electrostimulation. If the electrodes are not in contact with the skin (N in S112), the controller 30a discontinues electrostimulation and terminates the process S100. In essence, the controller 30a discontinues electrostimulation once the electrodes are detached from the body 2, even if the period of time T2 has not elapsed. If the period of time T2 has elapsed (Y in S110), the controller 30a discontinues electrostimulation and terminates the process S100.

The process S100 is by way of example only and other steps may be added, or steps may be deleted or modified, or the sequence of steps may be switched.

A description will now be given of the advantage and benefit of the muscle electrostimulation device 10 according to the first embodiment configured as described above.

The muscle electrostimulation device 10 according to the first embodiment includes a main body 20 having a contact surface 10b fitted around the abdomen 3. The contact surface 10b extends in the X direction encircling the abdomen 3 and in the Y direction perpendicular to the X direction. The main body 20 includes the front part 22 having the front electrode unit 32 adapted to cause an electric current in the X direction to flow in the abdominal rectus muscle 4 and a pair of extensions 26 extending from respective sides of the front part 22 in the X direction and having side electrode units 36 adapted to cause an electric current in the X direction to flow in the oblique abdominal muscle 5. The front recesses 23 that recede in the Y direction are provided at positions in side portions 22b, 22c of the front part 22 along the Y direction that bisect the front part 22 in the X direction. According to this configuration, the main body 20 includes the front part 22 and the extensions 26 so that it is possible to train the abdominal rectus muscle 4 and the oblique abdominal muscle 5 at the same time and reduce the effort of wearing the device on the body. By providing the front recesses 23, it is possible to identify the central position of the main body 20 by a touch at the fingertip as well as visually. It is therefore easy to suitably position the device on the body 2.

The main body 20 of the muscle electrostimulation device 10 according to the first embodiment is provided with the leads 37 electrically connected to the front electrode unit 32 or the side electrode units 36. The leads 37 have curved portions 37h curved inward in the Y direction to conform to the front recesses 23. According to this configuration, provision of the curved portions 37h secures a suitable distance between the leads 37 and the outer edge of the second base member 15 so that the front recesses 23 of a desired shape can be implemented.

In the muscle electrostimulation device 10 according to the first embodiment, the portion of the main body 20 provided with the front recesses 23 are provided with a thick portion 24g formed to have a larger thickness than elsewhere. According to this configuration, the strength of the front recesses 23 is improved, troubles such as a crack are minimized, and the durability is improved.

In the muscle electrostimulation device 10 according to the first embodiment, the dimension of the pair of extensions in the Y direction is configured to be smaller than the dimension of the front part 22 in the Y direction. According to this configuration, the width of the extensions 26 is reduced so that it is possible to accommodate the extensions 26 in a range without any bone between the costal bone and the pelvis and to cause an electric current to flow in the oblique abdominal muscle 5 effectively.

In the muscle electrostimulation device 10 according to the first embodiment, the front electrode unit 32 includes the electrode group 32b and the electrode group 32c spaced apart in the X direction by the distance X6, the side electrode units 36 include the first electrode 36b and the second electrode 36c spaced apart in the X direction by the distance X9, and the distance X9 is configured to be larger than the distance X6. According to this configuration, it is ensured that an electric current flows in a wider range in the oblique abdominal muscle 5 than when the distance X9 is smaller than the distance X6 so that the oblique abdominal muscle 5 can be trained effectively. By securing a large distance between the electrodes, an electric current can flow at a depth. It is therefore expected that the muscle is stimulated effectively.

In the muscle electrostimulation device 10 according to the first embodiment, the polarity of the voltages applied to the front electrode unit 32 and the side electrode units 36 is defined such that the electric current flowing in the abdominal rectus muscle 4 flows in a direction opposite to the electric current flowing in the oblique abdominal muscle 5. According to this configuration, an invalid current flowing between the electrodes of the front electrode unit 32 toward the side electrode units 36 and the electrodes of the side electrode units 36 toward the front electrode unit 32 can be reduced as compared to the case where the currents flow in the same direction.

In the muscle electrostimulation device 10 according to the first embodiment, the boundary recesses 25 that recede in the Y direction are provided on respective sides of the main body 20 along the Y direction at respective boundaries between the front part 22 and the pair of extensions 26. According to this configuration, it is easier for the extensions 26 to be twisted and the user can feel more comfortable wearing the device on the abdomen 3 than when the boundary recesses 25 are not provided.

In the muscle electrostimulation device according to the first embodiment, the main body 20 is provided with the leads 37 electrically connected to the side electrode units 36, and the leads 37 have curved portions 37h curved inward in the Y direction to conform to the boundary recesses 25. According to this configuration, provision of the curved portions 37h secures a suitable distance between the leads 37 and the outer edge of the second base member 15 so that the boundary recesses 25 of a desired shape can be implemented.

In the muscle electrostimulation device 10 according to the first embodiment, the portion of the main body 20 provided with the boundary recesses 25 is provided with a thick portion 25g formed to have a larger thickness than elsewhere. According to this configuration, the strength of the boundary recesses 25 is improved, troubles such as a crack in the boundary recesses 25 are minimized, and the durability of the extensions 26 against a twist is improved.

In the muscle electrostimulation device 10 according to the first embodiment, the main body 20 includes the first base member 14, the second base member 15, and the cover member 16. The first base member 14 has an opening 14b that exposes the front electrode unit 32 and the side electrode units 36. The second base member 15 is provided with the front electrode unit 32 and the side electrode units 36. The cover member 16 is built on the side of the second base member 15 opposite to the first base member 14 and covers the front part 22 and the pair of extensions 26 so as to integrate the front part 22 and the pair of extensions 26. According to this configuration, the durability of the leads 37 is improved as compared to the case where the first base member 14 is not provided.

In the muscle electrostimulation device 10 according to the first embodiment, the first base member 14 and the second base member 15 are made of a polyethylene terephthalate-based resin and the cover member 16 is made of a resin material harder than the second base member 15. According to this configuration, the main body 20 is imparted with desired rigidity as compared to the case where the cover member is too soft. Once the front part 22 is positioned, the extensions 26 are also substantially positioned so that the ease of wearing the device on the abdomen 3 is improved.

In the muscle electrostimulation device 10 according to the first embodiment, the cover member 16 has the edge cover portion 16c that covers the outer edges 14e, 15e of the first base member 14 and the second base member 15. According to this configuration, the edge cover portion 16c covers the outer edges 14e, 15e so that the area of contact between the main body 20 and the body 2 is increased and the user can feel more comfortable than otherwise. The rigidity of the main body 20 is improved as compared to the case where the edge cover portion 16c is not provided.

In the muscle electrostimulation device 10 according to the first embodiment, the pair of belt joints 42 for attachment of the belt member 50 for securing the main body 20 looped around the abdomen 3 are provided in the pair of extensions 26. According to this configuration, it is easier to join the belts of the belt member 50 than when the belt joints 42 are not provided. By guiding the belt member 50 joined to the extensions 26 past the back of the abdomen 3, the side electrode units 36 are prevented from being elevated from the abdomen 3.

In the muscle electrostimulation device 10 according to the first embodiment, the main body 20 is provided with the control unit 30 for supplying a voltage to the front electrode unit 32 and the pair of side electrode units 36. The control unit 30 is configured to switch between the independent control mode for controlling supplied voltages independently and the concurrent control mode for controlling supplied voltages concurrently. According to this configuration, the intensity of training one of the abdominal rectus muscle 4 and the oblique abdominal muscle 5 is regulated by using the independent control mode, and the abdominal rectus muscle 4 and the oblique abdominal muscle 5 can be trained in coordination by using the concurrent control mode. In essence, a suitable intensity of training can be set in accordance with the user's situation.

### [Second embodiment]

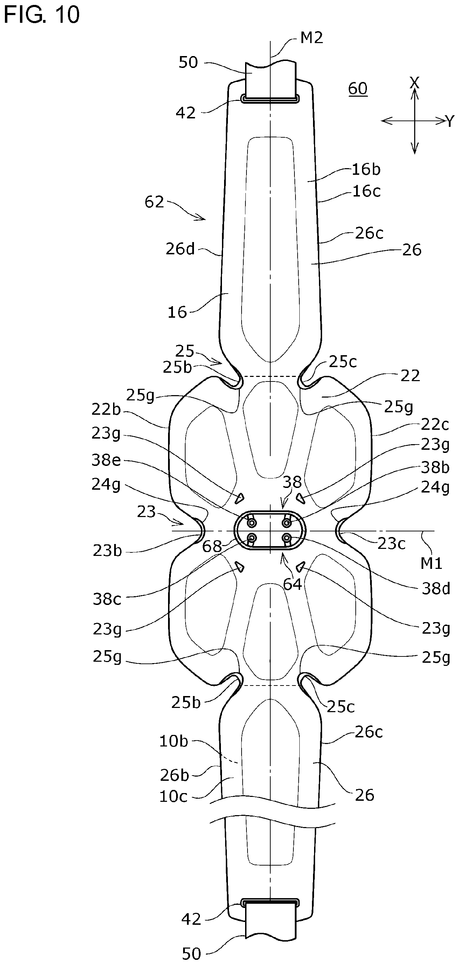

A description will be given of a muscle electrostimulation device 60 according to the second embodiment with reference to Figs. 10-12. In the drawings and description of the second embodiment, constituting elements and members identical or equivalent to those of the first embodiment shall be denoted by the same reference numerals. Duplicative explanations are omitted appropriately and features different from those of the first embodiment will be highlighted.

The control unit 30 of the muscle electrostimulation device 10 according to the first embodiment is described as being joined to the main body 20 in an integrated manner. The invention is non-limiting as to the feature. For example, the main body may not be provided with a control unit and the control unit may be configured as a separate device. The control unit may be configured to be detachably mounted on an attachment mechanism provided in the main body.

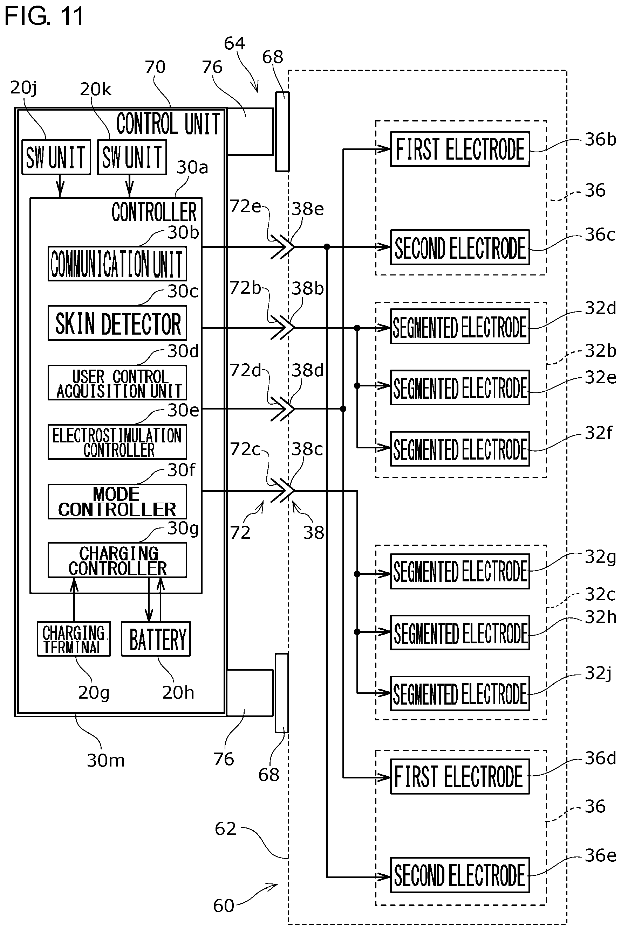

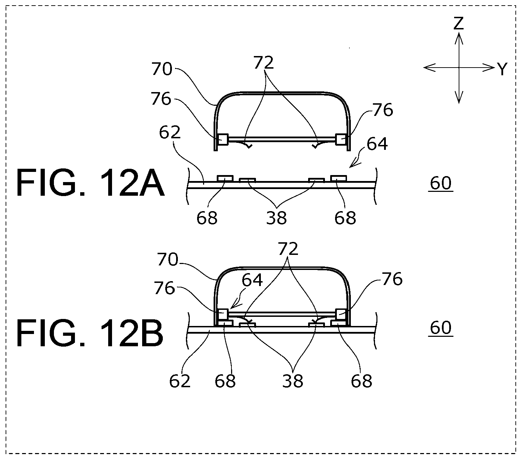

Fig. 10 is a front view of a main body 62 of the muscle electrostimulation device 60 according to the second embodiment. Fig. 11 is a block diagram showing features of the muscle electrostimulation device 60. Figs. 12A and 12B are side views schematically showing the vicinity of an attachment mechanism 64 of the muscle electrostimulation device 60. Figs. 12A and 12B show an example of the control unit 70 that can be attached to the muscle electrostimulation device 60. The muscle electrostimulation device 60 according to the second embodiment is not provided with a control unit in the main body 62 and is provided with the attachment mechanism 64 and power receiving terminals 38. The control unit 70 is configured as a device separate from the main body 62 and is attached to the main body 62 by the attachment mechanism 64. The control unit 70 is provided with power feeding terminals 72. When the control unit 70 is mounted to the main body 62, the power feeding terminals 72 come into contact with the power receiving terminals 38 and supply electric power for electrostimulation to the power receiving terminals 38.

The main body 62 of the second embodiment corresponds to the main body 20 of the first embodiment and is provided with all features and characteristics other than the control unit 30. The control unit 70 supplies a voltage to the front electrode unit 32 and the pair of side electrode units 36. The control unit 70 corresponds to the control unit 30 of the first embodiment and is provided with all features and characteristics of the control unit 30.

### (Attachment mechanism)

The main body 62 is provided with the attachment mechanism 64 and the power receiving terminals 38. The attachment mechanism 64 is a mechanism for detachably attaching the control unit 70 to the main body 62 . The attachment mechanism 64 may be configured such that the control unit 70 can be attached or detached using a tool. It is desired that the control unit 70 can be attached or detached manually without using a tool. A linking mechanism based on any of various principles such as that of general-purpose fasteners may be employed to configure the attachment mechanism 64 in the main body 62. One example of the attachment mechanism 64 that can be employed is a mechanism using magnetic attraction, a mechanism including a hook and a recess engageable with the hook, a mechanism including an external thread and an internal thread, a mechanism including a hook-and-loop fastener, and a mechanism including a snap button.

In the attachment mechanism 64 of this example, the control unit 70 is provided with a magnet 76, and the main body 62 is provided with a magnetic member 68 that attracts the magnetic pole of the magnet 76 magnetically. The magnetic attraction between the magnetic pole of the magnet 76 and the magnetic member 68 maintains the control unit 70 in the main body 62. When a force beyond the attraction of the magnet 76 is exerted on the control unit 70, the control unit 70 is removed from the main body 62. Fig. 12A shows the muscle electrostimulation device 60 in which the control unit 70 is not attached. Fig. 12B shows the muscle electrostimulation device 60 in which the control unit 70 is attached.

### (Power receiving terminals)

The power receiving terminals 38 include four terminals 38b, 38c, 38d, 38e arranged in the circumferential direction near the center of the main body 62. As in the first embodiment, the terminals 38b, 38c are connected to the electrode groups 32b, 32c, the terminal 38d is connected to the first electrodes 36b, 36d, and the terminal 38e is connected to the second electrodes 36c, 36e. The surface of the terminals 38b, 38c, 38d, 38e may be treated to prevent rust on the electrode surface. For example, the terminals may be plated with noble metal. The power receiving terminals 38 may be configured as a spring member in order to stabilize the pressure of contact with the power feeding terminals 72.

### (Power feeding terminals)

The power feeding terminals 72 include terminals 72b, 72c, 72d, 72e arranged at positions aligned with the terminals 38b, 38c, 38d, 38e of the power receiving terminals 38. For example, the terminals 72b, 72c, 72d, 72e may be configured as a spring member. The terminals 72b, 72c, 72d, 72e supply electric power for electrostimulation to the terminals 38b, 28c, 38d, 38e.

The muscle electrostimulation device 60 according to the second embodiment configured as described above operates similarly to the muscle electrostimulation device 10 according to the first embodiment when the control unit 70 is attached.

The muscle electrostimulation device 60 according to the second embodiment provides the same advantage and benefit as those of the muscle electrostimulation device 10 according to the first embodiment. In addition, the muscle electrostimulation device 60 according to the second embodiment provides the following advantage and benefit.

The main body 62 of the muscle electrostimulation device 60 according to the second embodiment is provided with the attachment mechanism 64 for detachably attaching the control unit 70, which supplies a voltage to the front electrode unit 32 and the pair of side electrode units 36. According to this configuration, one control unit may be switchably used in different types of main bodies. Further, if the main body goes out of order, only the main body may be replaced. This is more economical and saves more resources than when the control unit is not replaceable.

The embodiments are intended to be illustrative only and it will be understood to those skilled in the art that variations and modifications are possible. The description in this specification and the drawings shall be treated to serve illustrative purposes and shall not limit the scope of the invention as defined in the appended claims.

A description will now be given of variations. In the drawings and description of the variations, constituting elements and members identical or equivalent to those of the embodiments shall be denoted by the same reference numerals. Duplicative explanations are omitted appropriately and features different from those of the embodiments will be highlighted.

### (First variation)

In the embodiments, the belt member 50 is described as being used to fit the muscle electrostimulation device to the abdomen 3 but the embodiments are non-limiting as to the feature. The stickiness of a gel pad may be used to attach the muscle electrostimulation device to the abdomen 3.

### (Second variation)

In the embodiments, the remote controller 12 is described as being provided with a touch panel capable of display and touch operations, but the embodiments are non-limiting as to the feature . An input for user control may be entered in the remote controller 12 using a user control device other than a touch panel, based on an image displayed on a display device other than a touch panel. The display means is exemplified by a liquid display device and the user control means is exemplified by a key switch such as a keyboard.

The variations described above provide the same advantage and benefit as the embodiments.

Arbitrary combinations of the embodiments and combinations of an embodiment and a variation will also be useful. A new embodiment created by a combination of embodiments will provide the combined advantages of the embodiment and the variation as combined.

## Claims

1. A muscle electrostimulation device (10) comprising:
a main body (20) having a contact surface (10b) for fitting around an abdomen (3), wherein
the contact surface (10b) extends in an X direction encircling the abdomen (3) and in a Y direction perpendicular to the X direction,
the main body (20) including:
a front part (22) having a front electrode unit (32) comprising a plurality of electrode groups (32b, 32c) spaced apart in the X direction to cause an electric current in the X direction in an abdominal rectus muscle (4); and
a pair of extensions (26) extending from respective sides of the front part (22) in the X direction and each having a respective side electrode unit (36) comprising a plurality of electrodes spaced apart in the X direction to cause an electric current in the X direction to flow in a respective oblique abdominal muscle (5), wherein:
boundary recesses (25) that recede in the Y direction are provided on respective sides of the main body (20) along the Y direction at respective boundaries between the front part (22) and the pair of extensions (26); and
front recesses (23) that recede in the Y direction are provided at positions in side portions (22b) of the front part (22) along the Y direction that bisect the front part (22) in the X direction.

2. The muscle electrostimulation device (10) according to claim 1, wherein
the main body (20) includes a first base member (14), a second base member (15), and a cover member (16),
the first base member has an opening (14b) that exposes the front electrode unit (32) and the side electrode units (36),
the second base member is provided with the front electrode unit (32) and the side electrode units (36), and
the cover member (16) is built on a side of the second base member (15) opposite to the first base member (14) and covers the front part (22) and the pair of extensions (26) so as to integrate the front part (22) and the pair of extensions (26) .

3. The muscle electrostimulation device (10) according to claim 2, wherein
the cover member (16) has an edge cover portion (16c) that covers outer edges (14e,15e) of the first base member (14) and the second base member (15).

## Patentansprüche

1. Muskelelektrostimulationsvorrichtung (10), umfassend:
einen Hauptkörper (20) mit einer Kontaktoberfläche (10b), so dass dieser um ein Abdomen (3) herum passt, wobei
die Kontaktoberfläche (10b) sich in einer X-Richtung, welche das Abdomen (3) umläuft, und in einer Y-Richtung senkrecht zu der X-Richtung erstreckt,
wobei der Hauptkörper (20) einschließt:
ein Frontteil (22) mit einer Frontelektrodeneinheit (32), die eine Vielzahl von Elektrodengruppen (32b, 32c) umfasst, die in der X-Richtung beabstandet sind,
um einen elektrischen Strom in der X-Richtung in einem geraden Bauchmuskel (4) zu bewirken; und
ein Paar Fortsätze (26), die sich von jeweiligen Seiten des Frontteils (22) in der X-Richtung erstrecken, und
von denen jeder eine jeweilige Seitenelektrodeneinheit (36) aufweist, die eine Vielzahl von Elektroden aufweist, die in der X-Richtung beabstandet sind, um zu bewirken, dass ein elektrischer Strom in der X-Richtung in einem jeweiligen schrägen Bauchmuskel (5) fließt, wobei:
Grenzaussparungen (25), die in der Y-Richtung eingerückt sind, auf jeweiligen Seiten des Hauptkörpers (20) entlang der Y-Richtung an jeweiligen Grenzen zwischen dem Frontteil (22) und dem Paar von Fortsätzen (26) bereitgestellt werden; und
Frontaussparungen (23), die in der Y-Richtung eingerückt sind, an Positionen in Seitenabschnitten (22b) des Frontteils (22) entlang der Y-Richtung bereitgestellt werden, welche das Frontteil (22) in der X-Richtung halbieren.

2. Muskelelektrostimulationsvorrichtung (10) nach Anspruch 1, wobei
der Hauptkörper (20) ein erstes Basiselement (14), ein zweites Basiselement (15) und ein Abdeckelement (16) einschließt,
das erste Basiselement eine Öffnung (14b) aufweist, welche die Frontelektrodeneinheit (32) und die Seitenelektrodeneinheiten (36) exponiert,
das zweite Basiselement mit der Frontelektrodeneinheit (32) und den Seitenelektrodeneinheiten (36) ausgestattet ist, und
das Abdeckelement (16) auf einer Seite des zweiten Basiselements (15) gegenüber dem ersten Basiselement (14) ausgebildet ist und das Frontteil (22) und das Paar von Fortsätzen (26) abdeckt, um so das Frontteil (22) und das Paar von Fortsätzen (26) zu integrieren.

3. Muskelelektrostimulationsvorrichtung (10) nach Anspruch 2, wobei
das Abdeckelement (16) einen Randabdeckabschnitt (16c) aufweist, der Außenränder (14e, 15e) des ersten Basiselements (14) und des zweiten Basiselements (15) abdeckt.

## Revendications

1. Dispositif d'électrostimulation musculaire (10), comprenant :
un corps principal (20) ayant une surface de contact (10b) destinée à aller autour d'un abdomen (3), dans lequel la surface de contact (10b) s'étend dans une direction X encerclant l'abdomen (3) et dans une direction Y perpendiculaire à la direction X,
le corps principal (20) incluant :
une partie avant (22) ayant une unité à électrodes avant (32) comprenant une pluralité de groupes d'électrode (32b, 32c) espacés les uns des autres dans la direction X pour causer un courant électrique dans la direction X dans un muscle droit abdominal (4) ; et
une paire d'extensions (26) s'étendant à partir de côtés respectifs de la partie avant (22) dans la direction X et chacune ayant une unité à électrodes latérale respective (36) comprenant une pluralité d'électrodes espacées les unes des autres dans la direction X pour causer la circulation d'un courant électrique dans la direction X dans un muscle abdominal oblique respectif (5), dans lequel :
des évidements de limite (25) qui sont en retrait dans la direction Y sont prévus sur des côtés respectifs du corps principal (20) le long de la direction Y à des limites respectives entre la partie avant (22) et la paire d'extensions (26) ; et
des évidements avant (23) qui sont en retrait dans la direction Y sont prévus à des positions dans des portions latérales (22b) de la partie avant (22) le long de la direction Y qui bissecte la partie avant (22) dans la direction X.

2. Dispositif d'électrostimulation musculaire (10) selon la revendication 1, dans lequel
le corps principal (20) inclut un premier élément de base (14), un second élément de base (15), et un élément de couverture (16),
le premier élément de base a une ouverture (14b) qui expose l'unité à électrodes avant (32) et l'unité à électrodes latérales (36),
le second élément de base est pourvu de l'unité à électrodes avant (32) et de l'unité à électrodes latérales (36), et
l'élément de couverture (16) est construit sur un côté du second élément de base (15) opposé au premier élément de base (14) et couvre la partie avant (22) et la paire d'extensions (26) afin d'intégrer la partie avant (22) et la paire d'extensions (26).

3. Dispositif d'électrostimulation musculaire (10) selon la revendication 2, dans lequel
l'élément de couverture (16) a une portion de couverture de bords (16c) qui couvre des bords extérieurs (14e, 15e) du premier élément de base (14) et du second élément de base (15).
